# EUROPÄISCHE PATENTSCHRIFT

(11) **EP 1 372 521 B1**
(45) Veröffentlichungstag und Bekanntmachung des Hinweises auf die Patenterteilung: **18.02.2009**
(21) Anmeldenummer: 01988074.9
(22) Anmeldetag: 22.12.2001
(51) Int. Cl.: A61C 13/00, A61K 6/027, C25D 1/14

(54) **VERFAHREN ZUR HERSTELLUNG VON VOLLKERAMISCHEN DENTALFORMTEILEN**
METHOD FOR PRODUCING ALL-CERAMIC DENTAL DEVICES
PROCEDE DE PRODUCTION DE PIECES MOULEES DENTAIRES EN CERAMIQUE PLEINE

(30) Priorität: 26.03.2001 DE 10115820
(43) Veröffentlichungstag der Anmeldung: 02.01.2004
(73) Patentinhaber: Wieland Dental + Technik GmbH & Co. KG, 75179 Pforzheim (DE)
(72) Erfinder: BURGER, Goran, 75179 Pforhheim (DE); KNOLL, Stefan, 70195 Stuttgart (DE); LAUBERSHEIMER, Jürgen, 76307 Karlsbad (DE)
(74) Vertreter: Patentanwälte Ruff, Wilhelm, Beier, Dauster & Partner
(86) Internationale Anmeldenummer: PCT/EP2001/015295
(87) Internationale Veröffentlichungsnummer: WO 2002/076321

(56) Entgegenhaltungen:
- EP-A2- 0 781 530
- EP-B1- 0 241 384
- WO-A1-99/50480
- WO-A1-99/52467

## Beschreibung

Die Erfindung betrifft zunächst ein Verfahren zur Herstellung von zwei- oder mehrgliedrigen vollkeramischen Dentalformteilen, insbesondere von Gerüstelementen für Brücken und dergleichen.

Schon immer war Keramik oder "Porzellan" ein attraktiver Werkstoff, um Zähne mit sehr zahnähnlichem Aussehen in Form und Farbe nachzubilden. Keramik ist ein chemisch beständiger, korrosionsfester und biokompatibler Werkstoff, der zudem noch, in schier unendlicher Menge in mineralischer Form verfügbar und somit preiswert ist. Aus diesem Werkstoff ist mit zahntechnischen Mitteln individueller Zahnersatz einfach und reproduzierbar herzustellen, so dass der Durchbruch des Werkstoffes "Dentalkeramik" eingetreten ist.

Um die einzige Schwäche dieses Werkstoffes, die Sprödigkeit, zu umgehen, wird zahntechnisch gefertigter Zahnersatz in der Regel schon seit langem als klassischer Werkstoff-Verbund hergestellt, z.B. als sogenannte Metallkeramik. Eine metallkeramische Krone oder Brücke besteht aus einem metallischen Gerüst bzw. Unterbau und einer der Zahnform nachempfundenen sogenannten Verblendung aus Dentalkeramik. Der Unterbau wird beim Einsetzen des Zahnersatzes direkt auf dem nach der zahnärztlichen Präparation verbleibenden Restzahn befestigt und wird oft als (Schutz-) Käppchen bezeichnet. Je nachdem, aus welchem Material bzw. aus welcher Legierung die Käppchen bestehen und je nach Herstellungsverfahren (Giessen, Galvanoforming-Verfahren, d. h. galvanische Abscheidung), können Probleme in Form von Korrosion und daraus resultierende Verfärbungen, Körperunverträglichkeiten u.a.m. entstehen. Deshalb wurden in den letzten Jahren zunehmend Systeme entwickelt, die vergleichbare Unterkonstruktionen aus keramischen Materialien herstellen und zahntechnisch weiterverarbeiten können.

Es gibt bereits mehrere funktionierende Systeme auf dem Dentalmarkt. So werden die Keramik-Käppchen beispielsweise durch manuelles Auftragen eines Schlickers auf einen Modellstumpf, anschließendem Sinterbrand sowie nachfolgender Infiltration mit Spezialglas (VITA In-Ceram) oder durch einen Pressvorgang unter Temperatureinwirkung (Empress, Fa. IVOCLAR) hergestellt. Es gibt auch Systeme, bei denen die Käppchen aus gesinterten oder vorgesinterten Keramikblöcken digital gefräst werden (DCS-System, CEREC usw.). Allen solchen sogenannten Vollkeramik-Systemen ist jedoch gemeinsam, dass sie die Passgenauigkeit metallischer Körper auf dem Restzahn, ob letztere nun gegossen sind oder durch galvanische Prozesse entstehen, in der Regel nicht erreichen. Zudem sind diese Systeme in der Anschaffung meist sehr teuer.

Die mangelnde Passgenauigkeit existierender Vollkeramik-Systeme ergibt sich hauptsächlich durch die verwendeten Formgebungsverfahren. Bei der Herstellung metallischer Käppchen wird gegossen oder galvanisiert, so dass sich das Metall in geschmolzener bzw. gelöster Form optimal der Stumpfgeometrie anpassen kann. Dagegen muss z. B. bei CADCAM-gestützten Vollkeramikverfahren nach einem digital aufgenommenen Datensatz aus festem Material spanabhebend gefräst werden. Das Scannen des Zahnstupfes und das Fräsen können aber, je nach der digitalen Auflösung der Systemkomponenten, bereits Ungenauigkeiten enthalten.

Eine weitere grundsätzliche Schwierigkeit bei allen existierenden oder zukünftigen Systemen zur Herstellung vollkeramischen Zahnersatzes aus gesinterten keramischen Werkstoffen hinsichtlich der Passgenauigkeit der fertigen Teile ist der keramische Schrumpf, also die mit dem verdichtenden Sinterprozess einhergehende Volumenschwindung keramischer Formteile. Dieser Sinterschrumpf lässt sich zwar innerhalb gewisser Grenzen reduzieren, aber nicht völlig vermeiden. Deshalb wird der mit dem Sinterschritt verbundene Sinterschrumpf beispielsweise indirekt dadurch vermieden, dass man bereits gesinterte Keramik (CADCAM-Verfahren, s. o.) verarbeitet oder versucht, auf andere Art und Weise ein porenfreies Feststoffgefüge zu erreichen (Glasinfiltration der weichen, porösen Keramik-Käppchen beim lnCeram-Verfahren, s. o.). Auch bei der elektrophoretischen Abscheidung von Keramikpartikeln muss das erhaltene keramische Formteil anschließend gesintert werden, so dass sich auch hier das geschilderte Problem des Sinterschrumpfes zeigt.

Die geschilderten Probleme stellen sich bei zwei- oder mehrgliedrigen vollkeramischen Dentalformteilen in besonderer Weise. Dies liegt unter anderem daran, dass solche Dentalformteile wie beispielsweise Brücken größere Abmessungen aufweisen, höheren mechanischen Belastungen ausgesetzt sind und häufig höhere Passgenauigkeiten erfordern. Diese Probleme führen dazu, dass beispielsweise Presskeramiken, bei denen mit Glaskeramik gearbeitet wird, oder glasinfiltrierte Oxidkeramiken (wie oben diskutiert) zur Herstellung von Brückenkonstruktionen nur bedingt oder gar nicht geeignet sind. Dies gilt vor allem für mehrspännige Brücken im Seitenzahngebiet.

Die EP-A-0781530 offenbart ein Verfahren zur Herstellung von Zahnkronen und/oder Zahnbrücken mit mit einem Geschiebe verbundenen Brückengliedern, wobei Verbinder oder Retentionsteile auf mindestens einem Zahnstumpf befestigt und von einer keramischen Masse abgedeckt sind. Ferner ist die Zahnkrone oder Zahnbrücke mit einem vorgefertigten keramischen Verstärkungselement ausgestattet, das als Armierung wirkt.

In der EP-B-0241384 ist erwähnt, dass bei einer Fertigung von Dentalteilen aus Keramikmaterial Modellmaterialien mit einer geringen linearen Expansion zwischen 0,1 % und 0,4 % verwendet werden können. Es ist jedoch nicht angesprochen, durch solche Expansionen den beim Sintern des erhaltenen Grünkörpers eintretenden Sinterschrumpf vollständig zu kompensieren. Bei dieser Druckschrift werden die im Grünkörper vorhandenen Poren durch Infiltration mit Glas verschlossen und dadurch der Sinterschrumpf verhindert. Im übrigen geht die EP-B-0241384 lediglich von üblichen Standardverfahren zum Aufbringen der Keramikpartikel auf das Modell aus, nämlich dem Eintauchen des Modells in einen Schlicker und Einstellung der Schichtdicke durch mechanische Hilfsmittel wie Pinsel, Spatel u.dgl.. Andere Auftragverfahren sind nicht erwähnt.

Auch in der WO 99/50480 A1 wird der Sinterschrumpf durch die Infiltration mit Glas kompensiert.

Die Erfindung stellt sich deshalb die Aufgabe, die geschilderten und weitere Nachteile des Standes der Technik bei der Herstellung von zwei- oder mehrgliedrigen vollkeramischen Dentalformteilen mindestens teilweise zu vermeiden. Dabei sollen insbesondere die hohe Festigkeit und Bruchzähigkeit einer gesinterten Oxidkeramik für zwei- oder mehrgliedrige vollkeramische Dentalformteile nutzbar gemacht werden. Außerdem soll vorzugsweise die Herstellung solcher Dentalformteile weitgehend vereinfacht werden. Schließlich sollen insbesondere hohe Passgenauigkeiten erzielt werden, unter Vermeidung der nachteiligen Effekte des geschilderten Sinterschrumpfes.

Diese Aufgabe wird gelöst durch das Verfahren mit den Merkmalen des Anspruchs 1. Bevorzugte Ausführungen des erfindungsgemäßen Verfahrens sind in den abhängigen Ansprüchen 2 bis 17 dargestellt. Anspruch 18 betrifft und umfasst eine Vorrichtung zur elektrophoretischen Abscheidung von zwei- oder mehrgliedrigen erfindungsgemäßen vollkeramischen Dentalformteilen. Bevorzugte Ausführungsformen dieser Vorrichtung sind in den abhängigen Ansprüchen 19 und 20 beschrieben. Der Wortlaut sämtlicher Ansprüche wird hiermit durch Bezugnahme zum Inhalt dieser Beschreibung gemacht.

Zum besseren Verständnis der Erfindung sei im folgenden kurz die Herstellung und Weiterverarbeitung zahntechnischer Modelle erläutert. Der Zahn oder die Zähne, die mit einem Dentalformteil, z. B. Brücke oder dergleichen, versehen werden sollen, werden vom Zahnarzt in bekannter Weise präpariert. Auch ein lmplantataufbautell kann als Ausgangspunkt dienen. Von dieser Mundsituation nimmt der Zahnarzt einen Abdruck mit Hilfe eines aushärtenden Elastomermaterials. Hier kann es sich beispielsweise um einen Silikonkunststoff handeln. Der so erhaltene Abdruck stellt ein Negativmodell der vom Zahnarzt vorgenommen Präparation dar. Dieser Abdruck, d. h. das Negativmodell wird dem Zahntechniker übergeben, der diesen Abdruck mit Hilfe eines geeigneten Modellmaterials, meist einem sogenannten Dentalgips, ausgießt. Nach dem Abbinden des Gipses entsteht ein Positivmodell, das sogenannte Meistermodell, welches der Präparation des Zahnarztes exakt entspricht. Dieses Meistermodell wird üblicherweise als Vorlage zurückbehalten. Es wird dazu verwendet, ein oder mehrere Arbeitsmodelle herzustellen, die dann weiterverarbeitet werden. Die Herstellung des Arbeitsmodells erfolgt durch Duplieren, d. h. mit Hilfe eines Dupliermaterials, beispielsweise Silikonkunststoff, wird ein Negativmodell hergestellt, das dann wiederum mit Dentalgips ausgegossen wird. Auf diese Weise wird ein weiteres Positivmodell, nämlich das Arbeitsmodell erstellt.

Bei dem erfindungsgemäßen Verfahren zur Herstellung von zwei- oder mehrgliedrigen vollkeramischen Dentalformteilen wird so vorgegangen, dass von der Grundstruktur, für die das Dentalformteil vorgesehen ist, ein Modell hergestellt wird, dann mit Hilfe des Modells und einer Suspension keramischer Partikel ein keramischer Grünkörper gebildet wird, und dieser Grünkörper, gegebenenfalls nach Entfernen/Entformen von dem Modell, gesintert wird. Dabei sind die Abmessungen des Modells mindestens teilweise so gewählt, dass der beim Sintern des Grünkörpers eintretende Sinterschrumpf kompensiert wird, um dadurch die gewünschte Passung zwischen Dentalformteil und Grundstruktur zu erreichen. Bei bevorzugten Ausführungen sind dabei im wesentlichen alle Abmessungen des Modells so gewählt, dass der Sinterschrumpf kompensiert wird.

Grundsätzlich sind verschiedene Möglichkeiten denkbar, den Sinterschrumpf durch die Abmessungen des Modells zu kompensieren. Insbesondere ist das Modell bei dem erfindungsgemäßen Verfahren zur Kompensation des Sinterschrumpfs aus einem Modellmaterial mit erhöhter linearer Abbindeexpansion gefertigt. Vorzugsweise handelt es sich bei einem solchen Modellmaterial um einen sogenannten Dentalgips.

Gemäß dem Stand der Technik war es für den Fachmann bisher selbstverständlich, ein Modellmaterial mit einer möglichst geringen Expansion beim Abbinden bzw. Aushärten zu verwenden. Nur auf diese Weise kann nämlich die geforderte Dimensionstreue zwischen Präparation des Zahnarztes auf der einen Seite und Meistermodell bzw. Arbeitsmodell auf der anderen Seite gewährleistet werden. Deshalb sind beispielsweise die Abbindeexpansionen von gängigen Modellmaterialien wie Dentalgipsen in der Regel sehr gering. Diese Abbindeexpansion lässt sich nach den bekannten Beziehungen der Dilatometrie als lineare Ausdehnung ΔI/I₀ oder Volumenausdehnung ΔV/V₀ nach üblichen Methoden bestimmen. Die lineare Ausdehnung bei handelsüblichen Dentalgipsen, d. h. die Längenänderung, die ein entsprechender Gipskörper beim Abbinden erfährt, liegt bei weniger als 0,3 %. Grundsätzlich werden möglichst geringe Werte angestrebt. So liegen die linearen Expansionswerte bei den häufig gebrauchten Superhartgipsen der sogenannten Klasse IV bei ≤ 0,15 %.

Das bei der Erfindung eingesetzte Modellmaterial für Dentalzwecke zeichnet sich dagegen dadurch aus, dass es beim Abbinden bzw. Aushärten eine lineare Expansion von mindestens 0,5 %, vorzugsweise von mindestens 1 % aufweist. Bevorzugte Werte für die lineare Expansion beim Abbinden/Aushärten liegen zwischen 4 % und 12 %. Innerhalb dieses Bereichs sind wiederum Werte zwischen 8 % und 10 % hervorzuheben.

Ein Modellmaterial wie es die Erfindung verwendet, widerspricht dem bisherigen Verständnis des Fachmanns völlig. Wie bereits erläutert, war es bisher das Ziel, Modellmaterialien mit einer möglichst geringen Expansion beim Abbinden/Aushärten zur Verfügung zu stellen. Die Erfindung setzt nun bewusst Modellmaterialien mit höheren Expansionen ein, um auf diese Weise den bei der Herstellung von vollkeramischen Dentalformteilen auftretenden Sinterschrumpf zu kompensieren. Wird nämlich das Meistermodell oder bevorzugt das Arbeitsmodell bewusst "überdimensioniert", so kann der Sinterschrumpf in Kauf genommen werden. Sind das Expansionsverhalten des Modellmaterials und das Sinterschrumpfverhalten der Keramik bekannt, so kann ein exakt dimensioniertes vollkeramisches Dentalformteil zur Verfügung gestellt werden.

Grundsätzlich kann das erfindungsgemäß eingesetzte Modellmaterial aus den unterschiedlichsten Substanzen, die auch organischer Natur sein können, bestehen. Bei bevorzugten Ausführungsformen der Erfindung besteht das Modellmaterial jedoch hauptsächlich und dabei insbesondere vollständig aus anorganischen Substanzen. Gegebenenfalls können Zusätze vorhanden sein, die die Abbindeexpansion oder sonstige chemische und physikalische Eigenschaften des Modellmaterials beeinflussen. Auch bei diesen Zusätzen handelt es sich vorzugsweise um anorganische Substanzen.

Es ist besonders bevorzugt, wenn das Modellmaterial vollständig oder hauptsächlich aus Gips besteht. In der Regel handelt es sich dann um sogenannte Dentalgipse, die den besonderen Anforderungen auf dem Dentalgebiet, beispielsweise bezüglich der Modellierfähigkeit und der sogenannten Zeichnungsgenauigkeit Rechnung tragen. Gips ist in der Summe seiner Eigenschaften für den Zahntechniker nach wie vor das Modellmaterial der Wahl. Bei exakter und produktgerechter Verarbeitung eignet sich Gips für alle Arten von Modellen in der Zahntechnik und deren Herstellung.

Feinpulvriger Dentalgips, chemisch CaSO₄ · ½ H₂O ("Calciumsulfat-Halbhydrat"), wird mit einer bestimmten Menge Wasser (H₂O) angemischt und zur Herstellung von Gipsduplikaten von Zähnen bzw. Gebissen verwendet. Der sich beim Anmischen bildende Gipsbrei wird in eine leicht entfernbare Form aus Dupliermaterial (meist Silikon) gefüllt, die dem Abdruck der Mundsituation entspricht. Die Masse bindet dann unter Reaktion mit dem Wasser zu CaSO₄ · 2 H₂O, dem Calciumsulfat-Dihydrat ab:

CaSO₄ · ½ H₂O + 1 ½ H₂O → CaSO₄ · 2 H₂O

Wie an der chemischen Formel abzulesen ist, wird ein Teil des zugegebenen Wassers beim Abbinden chemisch als sog. "Kristallwasser" gebunden. Beim Abbindevorgang verfestigt bzw. erhärtet der Gips. Es wird Wärme freigesetzt und der Prozess geht mit einer reproduzierbaren Expansion einher, die als lineare Ausdehnung ΔI/I oder als Volumenausdehnung ΔV/V bestimmbar ist. Diese Expansion ist bei den erfindungsgemäß verwendeten Dentalgipsen bewusst hoch eingestellt.

Bei detaillierter Betrachtung ist der Abbindevorgang eine Summe von Einzelprozessen. Durch die Mischung des trockenen Gipspulvers mit Wasser entsteht eine übersättigte Lösung von Calciumsulfat-Halbhydrat, das Wasser aufnimmt und zu Dihydrat wird. Ausgehend von Kristallisationskeimen wachsen durch Aufnahme weiterer Dihydratmoleküle sog. Cluster, die weiter zu Kristallen wachsen. Durch die Bildung neuer Keime sowie das ständige Wachsen der Dihydrat-Kristalle entsteht so langsam ein immer fester werdendes Netzwerk von sich gegenseitig verhakenden und durchdringenden Kristallen, dessen Volumen größer ist als die Summe der einzelnen Kristallvolumina. Dies äußert sich makroskopisch dadurch, dass der Gips beim Abbinden die bereits erwähnte (Volumen)Expansion erfährt. Zusätzlich wird Energie in Form von Wärme frei.

Wie bereits erwähnt, kann das verwendete Modellmaterial Zusätze enthalten, die insbesondere auf den Abbinde- bzw. Aushärtevorgang Einfluss nehmen. Derartige Zusätze beeinflussen Parameter wie die Expansion beim Abbinden/Aushärten, die Zeitdauer des Abbindens/Aushärtens, die Härte des erhaltenen Modells und dergleichen. Vorzugsweise handelt es sich bei den Zusätzen um anorganische Substanzen, insbesondere um Salze. So kann beispielsweise ein Zusatz von Kochsalz die Volumenexpansion von Dentalgipsen beim Abbinden erhöhen. Vorzugsweise werden jedoch Silikate als Zusatz zur Erhöhung der Volumenexpansion verwendet. Solche Silikate können beispielsweise in Form von Kieselsol eingesetzt werden. Es ist dabei möglich, die Silikate dem Gipspulver entweder direkt oder in Form silikathaltiger Anmischflüssigkeiten zuzugeben.

Die Expansion des Modells, dessen Herstellung von dem erfindungsgemäßen Verfahren umfasst ist, kann zusätzlich dadurch in gewünschter Weise erhöht werden, indem man das geformte Modell beim Abbinden/Aushärten über einen gewissen Zeitraum mindestens teilweise, vorzugsweise vollständig in eine Flüssigkeit, insbesondere ein Lösungsmittel eintaucht. Bei der Flüssigkeit handelt es sich vorzugsweise um die Flüssigkeit, mit der das Modellmaterial versetzt, insbesondere angerührt wird, um es in die für das Ausgießen der Form nötige breiige bzw. pastöse Form zu bringen. Im Falle der Verwendung von Dentalgips als Modellmaterial handelt es sich bei dieser Flüssigkeit üblicherweise um Wasser. In diesen Fällen lässt man das Gipsmaterial also dementsprechend unter Wasser abbinden.

Bei dem erfindungsgemäßen Verfahren ist es weiter bevorzugt, dass nach dem Abbinden/Aushärten erhaltene Modell mindestens teilweise zu trocknen. Dies geschieht üblicherweise durch einfaches Stehenlassen der Modelle an Luft, wobei üblicherweise ein Zeitraum zwischen 0,5 Stunden bis 3 Stunden ausreicht. Bei der Trocknung verdunstet das im Gips nicht als Kristallwasser chemisch gebundene Wasser. Der Trocknungsprozess kann durch Anwendung erhöhter Temperaturen unterstützt werden. Bei bevorzugten Ausführungsformen wird zum Trocknen der Modelle mindestens ein Mikrowellentrocknungsschritt angewendet.

Die Mikrowellentrocknung dauert in der Regel nur wenige Minuten und kann in einem haushaltsüblichen Mikrowellengerät vorgenommen werden.

Bei dem erfindungsgemäßen Verfahren wird dann eine Suspension keramischer Partikel, der sogenannte keramische Schlicker, auf das Modell, üblicherweise ein Arbeitsmodell, aufgebracht. Dieses Arbeitsmodell weist dementsprechend aufgrund der eingetretenen Volumenexpansion größere Abmessungen/Dimensionen auf als die vom Zahnarzt präparierte Grundstruktur im Mund. Dieses Arbeitsmodell besitzt dementsprechend üblicherweise auch größere Abmessungen/Dimensionen als das Meistermodell, das die Mundsituation exakt wiedergeben soll und zweckmäßigerweise nicht aus dem Modellmaterial hergestellt wird. Durch die größeren Abmessungen/Dimensionen des Arbeitsmodells, auf das der keramische Schlicker aufgebracht wird, wird der im Sinterschritt eintretende Sinterschrumpf bereits vorab berücksichtigt.

In diesem Zusammenhang sei erwähnt, dass das für das Aufbringen des keramischen Schlickers endgültig verwendete Arbeitsmodell erfindungsgemäß auch in mehreren Durchgängen hergestellt werden kann, je nachdem welches Modellmaterial eingesetzt wird. Auf diese Weise kann man sich den gewünschten größeren Abmessungen des Arbeitsmodells zur Kompensation des Sinterschrumpfes sukzessive annähern oder gegebenenfalls sogar verschiedene vollkeramische Formteile erstellen und deren Passung mit dem Meistermodell testen.

Das eingangs genannte Verfahren, bei dem ein Modell von der Grundstruktur hergestellt wird, dann ein keramischer Grünkörper mit Hilfe des Modells und einer Suspension keramischer Partikel gebildet wird, und dieser Grünkörper gesintert wird, insbesondere das bisher geschilderte Verfahren, kann vorzugsweise so ausgestaltet sein, dass bei der Bildung des Grünkörpers mindestens zwei Glieder des Dentalformteils in einem Arbeitsschritt gleichzeitig aus den keramischen Partikeln geformt werden. Insbesondere werden bei solchen Ausführungsformen alle Glieder des Dentalformteils gleichzeitig geformt. Diese später noch im einzelnen erläuterte Vorgehensweise hat den Vorteil, dass das Herstellungsverfahren insgesamt vereinfacht und beschleunigt wird.

Die keramische Suspension kann zur Herstellung des Grünkörpers erfindungsgemäß vorzugsweise durch elektrophoretische Abscheidung auf das Modell (Arbeitsmodell) aufgebracht werden. Die Grundlagen und die Durchführung einer solchen elektrophoretischen Abscheidung sind dem Fachmann bekannt. Dabei wird in Flüssigkeit dispergiertes, in diesem Fall keramisches Pulver, mit Hilfe eines elektrischen Feldes auf dem Modell als bereits vorverdichtete Schicht abgeschieden. Der auf diese Weise erhaltene keramische Körper, der sogenannte Grünkörper, wird, gegebenenfalls nach Trocknung und Entformung vom Modell, gesintert.

Bei der elektrophoretischen Formgebung wird das Modell der Mundsituation (Arbeitsmodell), das elektrisch, z. B. mit Leitsilberlack kontaktiert ist, als Elektrode in einen Stromkreis geschalten. Als Gegenelektrode dient beispielsweise eine Pt-Elektrode, deren Form je nach Form des Modells variiert werden kann, um ein hohes homogenes elektrisches Feld für das gesamte Modell zu erreichen.

Die Abscheidung des keramischen Schlickers auf das Arbeitsmodell erfolgt bei konstant gehaltener Spannung bzw. bei konstant gehaltenem Strom normalerweise über einen Zeitraum von 1 bis 60 Minuten. Typische Werte für die Abscheidespannung bzw. Abscheideströme liegen zwischen 1 und 100 V bzw. zwischen 1 und 500 mA. Die bei Verwendung der elektrophoretischen Abscheidung erhaltenen Gründichten sind üblicherweise größer als 70 %, vorzugsweise größer als 80 % der theoretischen Dichte. Die elektrophoretische Abscheidung kann gegebenenfalls automatisiert mit Hilfe eines entsprechenden Geräts erfolgen.

Die verwendeten Suspensionen keramischer Partikel sind Suspensionen dispergierter keramischer Pulver in geeigneten Lösungsmitteln. Wie erwähnt spricht man hier auch von sogenannten keramischen Schlickern. Als Lösungsmittel werden vorzugsweise polare Lösungsmittel verwendet, wobei es sich insbesondere um Wasser, Alkohole und deren Mischungen, oder Mischungen aus Wasser mit Alkoholen handelt. Vorzugsweise werden polare Lösungsmittel mit Dielektrizitätszahlen im Bereich zwischen 15 und 85, vorzugsweise im Bereich von 15 bis 20 verwendet.

Bei den keramischen Partikeln handelt es sich vorzugsweise um oxidkeramische Partikel, insbesondere um Aluminiumoxid (Al₂O₃)-Partikel und/oder Zirkonoxid (ZrO₂)-Partikel, oder deren Mischungen. Die Korngrößen der keramischen Partikel liegen vorzugsweise zwischen 1 nm und 100 µm, vorzugsweise zwischen 100 nm und 10 µm. Insbesondere sind die keramischen Partikel in der Suspension in einer Menge zwischen 10 und 90 Gewichtsprozent, vorzugsweise zwischen 40 und 60 Gewichtsprozent, bezogen auf das Gesamtgewicht der Suspension, enthalten.

Bei weiteren Ausführungsformen können innerhalb der Suspension mindestens 2 Fraktionen keramischer Partikel mit unterschiedlicher mittlerer Korngröße enthalten sein. Auf diese Weise kann erreicht werden, dass die Dichte des abgeschiedenen Grünkörpers erhöht wird, da die keramischen Partikel mit kleinerer mittlerer Korngröße die Zwischenräume zwischen den keramischen Partikeln mit größerer mittlerer Korngröße zumindest teilweise auffüllen. Bekanntermaßen folgt die Korngrößenverteilung einer Fraktion keramischer Partikel mit bestimmter mittlerer Korngröße einer Gauß-Verteilung. Dementsprechend sind bei den beschriebenen Ausführungen (um in diesem Bild zu bleiben) die zwei oder mehr Gauß-Kurven gegeneinander verschoben.

Üblicherweise sind noch Bindemittel Bestandteil der Suspension, wobei es sich vorzugsweise um mindestens einen Polyvinylalkohol oder um mindestens ein Polyvinylbutyral handelt. Solche Bindemittel dienen u. a. zur Verbesserung sowohl des Trocknungsverhaltens als auch der Festigkeiten der resultierenden Grünkörper. Die Bindemittel sind in der Suspension, bezogen auf deren Feststoffgehalt, vorzugsweise in Mengen zwischen 0,1 und 20 Gewichtsprozent, insbesondere zwischen 0,2 und 10 Gewichtsprozent enthalten.

Die verwendeten Schlicker zeichnen sich durch Viskositäten im Bereich von 1 mPa·s bis 50 mPa·s, vorzugsweise im Bereich von 3 bis 10 mPa·s bei einer Scherrate von 600 s-¹ aus.

Bei der Erfindung besitzen diejenigen Teile des Modells, die den Pfeilern, insbesondere den Pfeilerzähnen entsprechen, vorzugsweise eine stumpfartige, Form. Insbesondere besitzen diese Teile die Form eines Käppchens, das nach endgültiger Fertigstellung auf den zugehörigen Zahnstumpf oder eine entsprechende andere Grundstruktur aufgesetzt werden kann.

Weiter sind vorzugsweise diejenigen Teile des Modells, die den Seitengliedern oder den Brückengliedern des Modells entsprechen, als Hohlform ausgebildet. Dies bedeutet, dass bei solchen Ausführungen die keramische Suspension in diese Hohlform zur Herstellung des Grünkörpers eingebracht wird. Bei den Ausführungen mit Hohlform ist diese Hohlform an den den Pfeilern entsprechenden Teilen des Modells befestigt.

Bei den vorstehend beschriebenen bevorzugten Ausführungsformen sind die den Pfeilern entsprechenden Teile des Modells aus einem sogenannten Dentalgips gefertigt. Dabei handelt es sich vorzugsweise um den oben beschriebenen Dentalgips mit erhöhter linearer Abbindeexpansion. Weiterhin ist bei diesen Ausführungsformen die Hohlform vorzugsweise aus einem zahntechnisch modellierbaren Material gefertigt. Hier handelt es sich insbesondere um ein sogenanntes Dentalwachs. Auch bei der Hohlform kann der später eintretende Sinterschrumpf der eingebrachten Keramik durch entsprechend größer gewählte Abmessungen berücksichtigt sein.

Die bei den geschilderten Ausführungsformen vorgesehene Hohlform ist vorzugsweise dreischalig aufgebaut, wobei insbesondere eine Unterschale und zwei die Hohlform nach oben abschließende Seitenschalen vorgesehen sind. Ein solcher dreischaliger Aufbau erleichtert die Herstellung der Hohlform und damit des Modells, was im folgenden noch näher erläutert wird.

Die oben beschriebene Befestigung der Hohlform an den den Pfeilern entsprechenden Teilen des Modells erfolgt vorzugsweise mit Hilfe eines zahntechnisch modellierbaren Materials, insbesondere mit Hilfe von Dentalwachsen.

Ein erfindungsgemäß besonders bevorzugtes Verfahren umfasst zur Herstellung eines Gerüstelements für Brücken die folgenden Verfahrensschritte:

Zunächst wird ein vorgefertigtes Brückenglied oder das Modell eines solchen Brückenglieds zwischen zwei den Pfeilern entsprechenden (bereits vorgefertigten) Teilen des Modells platziert und einmodelliert. In einem nächsten Schritt wird das Brückenglied oder sein Modell einschließlich der Verbindungsstellen zu den Pfeilerteilen mit dem zahntechnisch modellierbaren Material (insbesondere Dentalwachs) einmodelliert, und zwar vorzugsweise von basal bis zum anatomischen Äquator. Die so erhaltene Modellation wird vom Modell wieder abgenommen und das Brückenglied bzw. sein Modell wird vom Restmodell/Arbeitsmodell entfernt. Dann wird die zunächst abgenommene Modellation wieder in der Ursprungsposition auf dem Restmodell platziert und fixiert, vorzugsweise mit Dentalwachs. Anschließend erfolgt eine Aufmodellation der fixierten Modellation zu einer vollständigen Hohlform, insbesondere mit Hilfe einer sogenannten Wachssonde. Schließlich wird mit Hilfe des so erhaltenen vollständigen Modells von Pfeilern und Brückenglied und einer Suspension keramischer Partikel der keramische Grünkörper gebildet.

Der im Zuge der erfindungsgemäßen Verfahrensführung hergestellte Grünkörper weist vorzugsweise eine durchschnittliche Schichtdicke von 0,2 bis 2 mm, insbesondere von 0,8 bis 1,2 mm auf. Dadurch können nach dem Sinterschritt die erwünschten Schichtdicken des vollkeramischen Formteils bereitgestellt werden.

Der keramische Grünkörper hat üblicherweise eine Gründichte von mindestens 70 % und wird bei den Temperaturen gesintert, die sich aus den verwendeten Keramikmaterialien ergeben. Vorzugsweise liegt die Sintertemperatur zwischen 1100 °C und 1700 °C, insbesondere zwischen 1500 °C und 1700 °C. Vorzugsweise beträgt die Sintertemperatur ca. 1600 °C.

Die Sinterzeit wird ebenfalls z. B. in Abhängigkeit von dem verwendeten Keramikmaterial gewählt. Hier sind bevorzugte Sinterzeiten zwischen 2 und 10 Stunden, insbesondere zwischen 2 und 6 Stunden zu nennen. Bei weiteren bevorzugten Ausführungsformen wird ca. 5 Stunden gesintert.

Um eine homogene Temperaturverteilung im Grünkörper zu erreichen, wird dieser allmählich auf die endgültige Sintertemperatur gebracht. Bevorzugte Aufheizraten betragen hier zwischen 1 und 20 °C/min, insbesondere zwischen 5 und 10 °C/min. Innerhalb des zuletzt genannten Bereichs sind Aufheizraten zwischen 5 und 7,5 °C/min weiter bevorzugt.

Vorzugsweise wird im Sinterschritt so vorgegangen, dass das Arbeitsmodell zusammen mit dem darauf abgeschiedenen Grünkörper bei Raumtemperatur an Luft getrocknet und dann anschließend in den Ofen überführt wird. Dort wird das Arbeitsmodell zusammen mit dem Grünkörper bis auf ca. 900 °C erhitzt, wobei hier eine vergleichsweise geringe Aufheizrate verwendet werden kann. Dieses Aufheizen kann stufenweise erfolgen, wobei Haltezeiten bei den entsprechenden Temperaturen vorgesehen sein können. Durch dieses Erhitzen wird der Grünkörper vorgesintert, wobei das Gipsmaterial des Arbeitsmaterials schrumpft, da der Gips sein Kristallwasser teilweise verliert. Dann wird das Arbeitsmodell zusammen mit dem Grünkörper kurz aus dem Ofen genommen und der Grünkörper vom Arbeitsmodell entformt. Dies geschieht leicht, da das Arbeitsmodell wie beschrieben geschrumpft ist. Dann wird der vorgesinterte Grünkörper, beispielsweise in Form eines Käppchens wieder in den Ofen gegeben. Dann wird der Ofen, vorzugsweise mit einer vergleichsweise hohen Aufheizrate auf die endgültige Sintertemperatur gebracht und das Formteil fertig gesintert.

Nach dem Sinterschritt werden vollkeramische Formteile mit Dichten von mehr als 90 % der theoretischen Dichte, vorzugsweise mehr als 95 % der theoretischen Dichte erhalten. Solche Vollkeramikteile, beispielsweise in Form einer Brückengrundstruktur, können dann in üblicher Weise wie ein Metallkäppchen mit Verblendkeramik versehen und gebrannt werden. Auf diese Weise entsteht der endgültige Zahnersatz, der beispielsweise in Form einer Brücke in den Mund des Patienten eingesetzt wird. Selbstverständlich kann der so herstellbare Zahnersatz auch auf dentale Suprakonstruktionen, wie beispielsweise Implantatteile aufgesetzt werden.

Schließlich umfasst die Erfindung noch eine Vorrichtung zur elektrophoretischen Abscheidung von zwei- oder mehrgliedrigen vollkeramischen Dentalformteilen, insbesondere von Gerüstelementen von Brücken und dergleichen. Diese Vorrichtung kann übliche Komponenten einer solchen Einrichtung zur elektrophoretischen Abscheidung umfassen wie Strom-/Spannungsquelle, Steuer- und Regeleinrichtungen, Abscheidebehältnis, Gegenelektrode, Kontaktierungseinrichtungen und dergleichen. Zusätzlich umfasst diese Vorrichtung erfindungsgemäß eine vorzugsweise anodisch schaltbare Hilfselektrode, die in Bezug auf das für die Abscheidung verwendete Modell in der Nähe einer Verbindungsstelle zwischen Pfeiler und Seitenglied/Brückenglied anordenbar ist. Bei bevorzugten Ausführungen dieser Vorrichtung sind zwei Hilfselektroden vorgegehen, die in der Nähe der beiden Verbindungsstellen zwischen Pfeilern und Brückenglied anordenbar sind. Dabei umschließen die Hilfselektroden die entsprechenden Verbindungsstellen mindestens teilweise.

Weitere Merkmale der Erfindung ergeben sich aus dem nachfolgenden Beispiel und der Figur in Verbindung mit den Unteransprüchen. Hierbei können die einzelnen Merkmale jeweils für sich allein oder zu mehreren in Kombination miteinander verwirklicht sein.

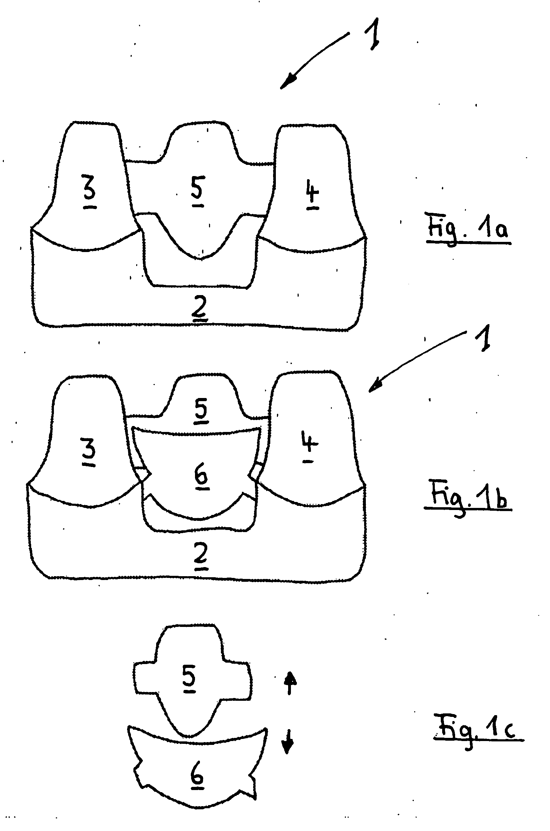

In den Zeichnungen zeigen Figur 1a bis Figur 1h die Verfahrensschritte bei einer bevorzugten Ausführungsform des erfindungsgemäßen Verfahrens.

### Beispiel

### 1. Herstellung von Suspensionen keramischer Partikel

### 1.1 Schlickerherstellung mit Aluminiumoxid-Pulver

Zur Herstellung eines Aluminiumoxid-Schlickers werden zunächst in 100 g deionisiertem Wasser 0,75 g Na₂P₄O₇·10 H₂O als Dispergierungsmittel zugegeben und durch Rühren mit Hilfe eines Magnetrührers gelöst. Anschließend erfolgt eine portionsweise Zugabe von 100 g Aluminiumoxid-Pulver mit einer Primärpartikelgröße (Partikelgröße in nicht-agglomeriertem Zustand) von ca. 0,6 µm unter ständigem Rühren. Die auf diese Weise erhaltene Suspension wird in einem weiteren Arbeitsschritt durch eine Ultraschallbehandlung mit 20 KHz und einer Leistung von 450 Watt 5 min lang dispergiert. Anschließend werden der Suspension 5 g Polyvinylalkohol zugegeben. Durch eine erneute Ultraschallbehandlung wird der resultierende Keramikschlicker homogenisiert.

Verwendete Chemikalien: Aluminiumoxidpulver CT 3000 SG (Fa. AL-COA; Fa. MERCK); Natriumpyrophosphat-Dekahydrat (Fa. RIEDEL DE HAEN); Polyvinylalkohol, Molekulargewicht 72.000 (Fa. CLARIANT).

### 1.2 Schlickerherstellung mit Zirkondioxid-Pulver

Es werden zu 100 g Ethanol, in dem zuvor 1 g Acetylaceton mit Hilfe eines Magnetrührers gelöst wurden, portionsweise 100 g ZirkonoxidPulver unter Rühren zugegeben. Die Primärpartikelgröße (Partikelgröße in nicht-agglomeriertem Zustand) des hierbei verwendeten Zirkondioxid-Pulvers liegt bei ca. 0,6 µm. Zur vollständigen Deagglomeration der hergestellten Suspension erfolgt anschließend eine 5 min andauernde UItraschallbehandlung. Der resultierenden Suspension werden 5 g. Polyvinylbutyral zugesetzt. Eine Homogenisierung des erhaltenen Schlickers erfolgt erneut durch eine Ultraschallbehandlung.

Verwendete Chemikalien: Zirkonoxidpulver SC 15 (Fa. MEL CHEMI-CALS); Acetylaceton (Fa. RIEDEL DE HAEN); Polyvinylbutyral, Molekulargewicht 70.000 (Fa. CLARINANT).

### 2. Herstellung eines vollkeramischen Gerüstelements für eine Brücke

Wie bereits in der Beschreibung geschildert, nimmt der Zahnarzt, üblicherweise nach Durchführung einer geeigneten Präparation, mit einem zeichnungsgenauen, aushärtende Elastomermaterial einen Abdruck der Mundsituation. Der gehärtete Andruck wird dann vom Zahntechniker mit einem Modellmaterial, meist Dentalgips, ausgegossen. Hierbei wird zweckmäßigerweise ein üblicher Dentalgips mit geringer linearer Abbindeexpansion verwendet. Nach dem Abbinden des Gipses entsteht das sogenannte Meistermodell. Durch das im folgenden noch beschriebene Duplieren dieses Meistermodells entsteht mindestens ein Arbeitsmodell, auf dem das vollkeramische Gerüstelement für eine Brücke nach dem erfindungsgemäßen Verfahren hergestellt werden kann.

Bei der geschilderten Vorgehensweise werden vom Zahntechniker vor dem Duplieren des Meistermodells üblicherweise die Präparationsgrenze freigelegt, die Brückenpfeilerstümpfe auf Kavitäten, Schleifrillen o. a. überprüft und letztere gegebenenfalls mit Dentalwachs oder einem geeignetem Kunststoff ausgeblockt. Dann erfolgt das bereits erwähnte Duplieren des Meistermodells. Dabei wird die vom Meistermodell mit Silikonkunststoff abgenommene Duplierform (Negativform) mit einem Spezialgips ausgegossen, dessen lineare Abbindeexpansion so eingestellt ist, dass sie den beim Sintern des später erhaltenen Grünkörpers eintretenden Sinterschrumpf kompensiert.

Das so erhaltene Arbeitsmodell 1 ist in Figur 1a dargestellt. Es besteht im wesentlichen aus der Basis 2 und den beiden Teilen 3 und 4, die den Pfeilerzähnen bzw. Pfeilerzahnstümpfen entsprechen. Dabei sei nochmals erwähnt, dass die Teile 3 und 4 des Arbeitsmodells 2 aufgrund der Verwendung des Dentalgipses mit erhöhter linearer Abbindeexpansion größere Abmessungen besitzen als die entsprechenden Teile des nicht dargestellten Meistermodells. Das Meistermodell gibt die Mundsituation von den Abmessungen her exakt wieder.

Auf das Arbeitsmodell 1 mit Basis 2 und Pfeilerzahnteilen 3 und 4 wird als nächstes ein Brückenglied 5 einmodelliert. Dieses Brückenglied 5 entspricht einer verkleinerten anatomischen Zahnform. Der spätere keramische Sinterschrumpf kann durch größere Abmessungen des Brückenglieds 5 bereits berücksichtigt werden. Die Einmodellation erfolgt mit einem Kunststoff, zum Beispiel dem sogenannten Pattern Resin der Firma GC, einem PMMA (Polymethylmethacrylat)-Kunststoff. Alternativ können auch vorgefertigte Brückenglieder aus Dentalwachs oder Kunststoff verwendet werden. In jedem Fall erfolgt die Einmodellation des Brückenglieds zwischen den Brückenpfeilerstümpfen unter Berücksichtigung der konkreten Mundsituation des Patienten. D.h. es werden die zu ersetzenden Zähne, das Restgebiß, die sogenannten Antagonisten, der sogenannte Sideshift und andere Faktoren berücksichtigt. Gleichzeitig werden die Verbindungsstellen zwischen Brückenglied und Brückenpfeilerstümpfen, die sogenannten Brückenverbinder, so groß wie möglich dimensioniert, um später eine hohe Stabilität des Gerüstelements zu gewährleisten.

Gemäß dem nächsten, in Figur 1b dargestellten Verfahrensschritt wird das Brückenglied samt Brückenverbinder von basal bis zum anatomischen Äquator mit einem rückstandslos verbrennbaren Dentalwachs zu einer Wachsmodellation 6 einmodelliert.

Diese Wachsmodellation 6 wird anschließend vom Brückenglied 5 abgenommen. Während das Brückenglied 5 eine Positivform darstellt, handelt es sich bei der Wachsmodellation 6 um eine Negativform. Nach Abnehmen der Wachsmodellation 6 vom Brückenglied 5 wird auch dieses Brückenglied 5 wieder von den Brückenpfeilerstümpfen 3 und 4 entfernt. Dies ist in Figur 1c durch die Pfeile angedeutet.

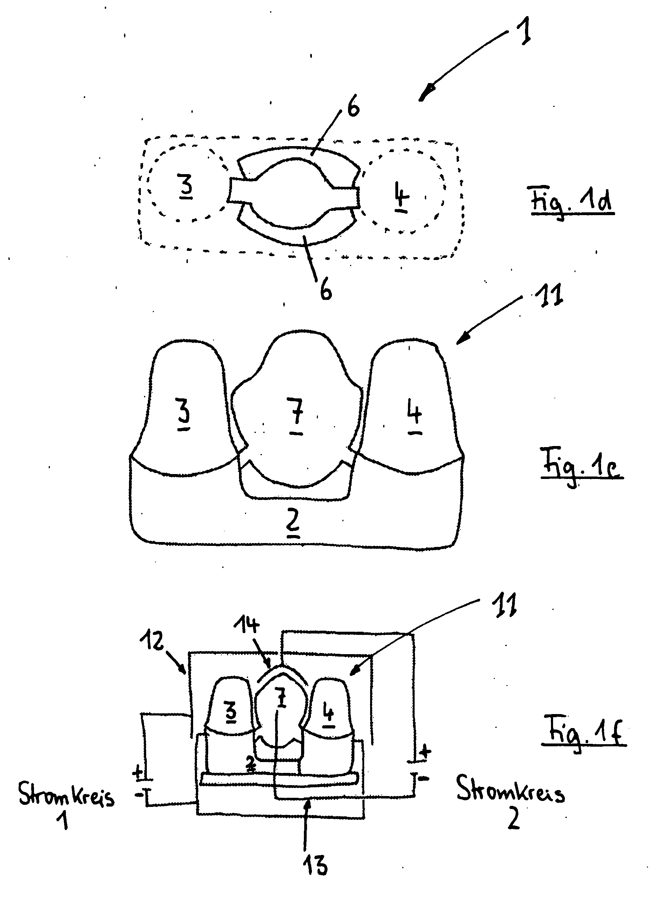

Nach Entfernen des Brückenglieds 5 von den Brückenpfeilerstümpfen 3 und 4 und damit vom Arbeitsmodell 1, wird die Wachsmodellation 6 (Negativform des Brückenglieds 5) wieder in ihre ursprüngliche Position zwischen die Brückenpfeilerstümpfe 3 und 4 auf dem Arbeitsmodell 1 aufgesetzt und mit Dentalwachs fixiert. Dieser Verfahrensschritt ist in Figur 1d dargestellt, und zwar zum besseren Verständnis im Blick von oben, d.h. von occlusal. Durch das Zurücksetzen der Wachsmodellation 6 auf das Arbeitsmodell 1 werden die Informationen über das Brückenglied 5 und die Brückenverbinder als Negativ auf das Arbeitsmodell 1 übertragen.

Als nächstes wird die endgültige Höhe des Brückenglieds 5 durch Komplettieren der Wachsformwände nach occlusal festgelegt, was in Figur 1e dargestellt ist. Dabei wird der occlusal nötige Rest des Brückenglieds mittels einer sogenannten Wachssonde aufmodelliert. Auf diese Weise erhält man zwischen den Brückenpfeilerstümpfen 3 und 4 des Arbeitsmodells 1 eine mit dem Arbeitsmodell 1 verbundene Hohlform 7. Diese Hohlform 7 entspricht in ihrer Form dem ursprünglich eingesetzten Brückenglied 5. Die Hohlform 7 ist in Richtung auf die Brückenpfeilerstümpfe 3 und 4 offen.

Die in Figur 1e dargestellte Situation zeigt das so erhaltene modifizierte Arbeitsmodell 11, von dem ausgehend ein keramischer Grünkörper hergestellt wird. Wie in Figur 1e dargestellt, besteht das modifizierte Arbeitsmodell 11 im wesentlichen aus der Basis 2, den den Brückenpfeilerstümpfen entsprechenden Teilen 3 und 4, so wie der zwischen den Teilen 3 und 4 angeordneten Hohlform 7, welche die Form des Brückenglieds als Negativform wiedergibt.

Das modifizierte Arbeitsmodell 11 wird im unteren Teil horizontal mit einer Diamanttrennschreibe durchtrennt, ohne dabei die Hohlform 7 zu verletzen. Der sich dadurch nach unten öffnende Spalt wird mit rückstandslos verbrennbarem Dentalwachs zugeschwemmt und das auf diese Weise bearbeitete modifizierte Arbeitsmodell 11 auf eine feste Unterlage, im vorliegenden Fall aus Aluminiumoxid aufgeklebt. Die Präparationsbereiche der den Pfeilerzähnen entsprechenden Teile 3 und 4 sowie die Innenseiten der Hohlform 7 werden mit Leitsilberlack eingestrichen und über Kupferzuleitungen/Kupferstäbe (nicht dargestellt) kontaktiert. Zweckmäßigerweise wird der Modellsockel (Unterlage) mit einem Lack abgedeckt, um auch im cervikalen Bereich definierte keramische Abscheidungen zu erhalten.

Die Situation beim elektrophoretischen Abscheiden der Keramik aus einem keramischen Schlicker, wie er in 1.1 und 1.2 beispielhaft hergestellt ist, ist in Figur 1f wiedergegeben. Demnach ist das modifizierte Arbeitsmodell 11, das sogenannte Abscheidemodell, als Kathode in einen Stromkreis geschaltet. Dabei werden in einem ersten Stromkreis (Stromkreis 1) die den Pfeilerzähnen entsprechenden Teile 3 und 4 des Arbeitsmodells 11 als Kathode (Minuspol) geschaltet und eine das gesamte Arbeitsmodell 11 deckelartig überspannende Elektrode 12 als Gegenelektrode (Anode, Pluspol). Weiter wird ein zweiter Stromkreis (Stromkreis 2) zur verstärkten Abscheidung im Bereich der Hohlform 7 ausgebildet, und zwar mit Hilfe einer direkt mit der Hohlform 7 kontaktierten weiteren Kathode 13 (Minuspol) und der die Hohlform 7 überspannenden Hilfselektrode 14 (Anode, Pluspol). Die anodisch geschaltete Hilfselektrode 14 ist dabei flächig nach Art eines Deckels oder einer Haube ausgestaltet, um eine möglichst großflächige Belegung der Hohlform 7 mit dem zugehörigen elektrischen Feld zu gewährleisten.

Die in Figur 1f dargestellte Anordnung einschließlich Arbeitsmodell 11 wird zur elektrophoretischen Abscheidung in den keramischen Schlicker getaucht und die beiden Stromkreise geschlossen. Im vorliegenden Fall wird an beide Stromkreise eine konstante Spannung von 14V angelegt. Daraus ergeben sich im Stromkreis 1 ein Anfangsstrom von 3,7 mA und im Stromkreis 2 ein Anfangsstrom von 2,5 mA. Dann wird gleichzeitig abgeschieden. Allerdings wird die elektrophoretische Abscheidung an den den Pfeilerzähnen entsprechenden Teilen 3 und 4 (Stromkreis 1) bereits nach 5 Minuten beendet, um keine zu großen Schichtdicken des aufgebrachten Keramikmaterials zu erhalten. Bei der Hohlform 7 (Stromkreis 2) wird 15 Minuten länger abgeschieden, d.h. über eine Gesamtzeit von 20 Minuten. Dies deshalb, damit an den Verbindungsstellen zwischen Brückenglied und Pfeilerzähnen möglichst dicke Keramikschichten abgeschieden werden. Am Ende der elektrophoretischen Abscheidung betragen die Ströme beim Stromkreis 1 noch 1,3 mA und beim Stromkreis 2 noch 0,9 mA. Wie bereits erwähnt werden die Spannungswerte über die gesamte Abscheidezeit jeweils bei 14 V konstant gehalten.

Nach der Abscheidung wird das Arbeitsmodell 11 samt Grünling entnommen und die Verbindung zwischen Kontaktierung (Kupferstab) und Grünling mittels eines feinkörnigen Diamantschleifers getrennt. Die Kupferstäbe werden entfernt und der cervikale Bereich wird mit einem Silikonpolierer bei geringer Drehzahl und geringem Anpressdruck ausgearbeitet.

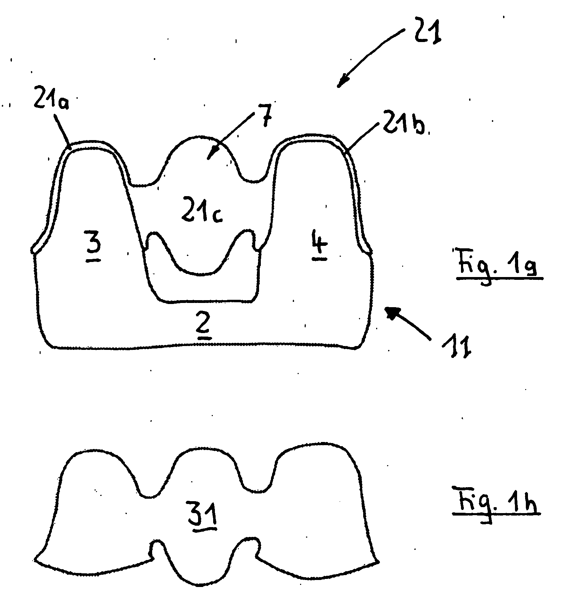

Die Situation nach dem elektrophoretischen Abscheiden ist in Figur 1g dargestellt. Figur 1g zeigt das modifizierte Arbeitsmodell (Abscheidemodell) 11 sowie den darauf abgeschiedenen Grünling bzw. Grünkörper 21 im Querschnitt. Das modifizierte Arbeitsmodell 11 besteht nach wie vor aus der Basis 2, den den Pfeilerzähnen/Pfeilerstümpfen entsprechenden Teilen 3 und 4 sowie dem aus Wachs modellierten Hohlkörper 7. Der einstückige Grünkörper 21 besteht aus den beiden kappenartigen Teilen 21 a und 21 b, die durch das im Inneren des Hohlkörpers 7 gebildete Keramikteil 21 c miteinander verbunden sind.

Um den Grünkörper 21 vom modifiziertem Arbeitsmodell 11 zu trennen, wird der Gesamtkörper aus Arbeitsmodell 11 und Grünkörper 21 einer ersten Temperaturbehandlung unterzogen. Bei dieser Temperaturbehandlung verbrennt zum einen das Wachs des Hohlkörpers 7 rückstandslos und zum anderen wird der Dentalgips des restlichen Arbeitsmodells aus Basis 2 und Teilen 3 und 4 entwässert. Die mit diesem Entwässern verbundene Volumenschwindung bewirkt die Ablösung des Grünkörpers 21 vom restlichen Arbeitsmodell. Bei dieser ersten Temperaturbehandlung wird im vorliegenden Fall eine Endtemperatur von 900 °C erreicht. Dabei wird zunächst mit einer Aufheizrate von 2 °C/min auf eine Temperatur von 70 °C aufgeheizt und diese Temperatur 30 Minuten lang gehalten. Dadurch schmilzt das Wachs des Hohlkörpers 7 aus. Anschließend wird mit einer Aufheizrate von 2 °C/min bis auf 600 °C aufgeheizt und direkt daran anschließend mit einer Aufheizrate von 5 °C/min bis auf die Endtemperatur von 900 °C. Hier beträgt die Haltezeit eine Stunde. Dann lässt man den Gesamtkörper abkühlen und nimmt den vorläufig wärmebehandelten Grünkörper 21 vom Restarbeitsmodell ab.

Der so erhaltene Grünkörper 21 wird anschließend in einem Sinterbrand zum endgültigen Brücken-Gerüstelement 31 dicht gesintert. Durch diesen Sinterbrand erhält das Brücken-Gerüstelement 31 seine endgültige Form und Festigkeit. Das entsprechende Gerüstelement 31 ist in Figur 1h nochmals dargestellt. Dabei wird im vorliegenden Beispiel so vorgegangen, dass zunächst mit einer Aufheizrate von 10 °C/min bis auf 900 °C aufgeheizt wird und unmittelbar daran anschließend mit einer Aufheizrate von 5 °C/min auf eine Endtemperatur von 1600 °C. Die Haltezeit bei 1600 °C beträgt 4 Stunden. Die sich daran anschließende Abkühlung erfolgt mit 5 °C Temperaturerniedrigung/min bis auf eine Temperatur von 900 °C. Anschließend lässt man den Sinterofen frei abkühlen bis auf Raumtemperatur.

Durch die Anwendung des erfindungsgemäßen Verfahrens besitzt das erfindungsgemäße Brücken-Gerüstelement 31 eine ausgezeichnete Passung mit den Teilen des Meistermodells, die den Pfeilerzahnstümpfen entsprechen. Zur Herstellung des endgültigen Zahnersatzes wird das Brücken-Gerüstelement 31 mit einer Dentalkeramik verblendet, die einen zur Keramik des Gerüstelements passenden Wärmeausdehnungskoeffzienten (WAK-Wert) besitzt. Wie erläutert wird erfindungsgemäß das gesamte Brückengerüst in einem Formgebungsschritt, hier elektrophoretischem Formgebungsschritt, hergestellt. Die Notwendigkeit, Brückenglied und Pfeilerzahnteile nachträglich miteinander zu verbinden, entfällt. Die elektrophoretische Abscheidung erfordert, wie ebenfalls dargestellt, keine aufwendige Ausrüstung, sondern lässt sich mit einer vergleichsweise einfachen Apparatur durchführen. Dies macht das erfindungsgemäße Verfahren wirtschaftlich sehr interessant.

## Patentansprüche

1. Verfahren zur Herstellung von zwei- oder mehrgliedrigen vollkeramischen Dentalformteilen, wie Gerüstelementen für Brücken (31) und dergleichen, bei dem von der Grundstruktur, für die das Dentalformteil vorgesehen ist, ein Modell (11) hergestellt wird, mit Hilfe des Modells (11) und einer Suspension keramischer Partikel ein keramischer Grünkörper (21) gebildet wird, und der keramische Grünkörper (21), ggf. nach Entfernen von dem Modell (11), gesintert wird, wobei der Grünkörper (21) aus den keramischen Partikeln durch elektrophoretische Abscheidung gebildet wird und die Abmessungen des Modells mindestens teilweise so gewählt sind, dass der beim Sintern des Grünkörpers eintretende Schrumpf kompensiert wird, um die gewünschte Passung zwischen Dentalformteil und Grundstruktur zu erreichen.

2. Verfahren nach Anspruch 1, **dadurch gekennzeichnet, dass** im wesentlichen alle Abmessungen des Modells (11) so gewählt sind, dass der Sinterschrumpf kompensiert wird.

3. Verfahren nach Anspruch 1 oder Anspruch 2, **dadurch gekennzeichnet, dass** das Modell zur Kompensation des Sinterschrumpfs aus einem Modellmaterial mit erhöhter linearer Abbindeexpansion gefertigt ist.

4. Verfahren nach Anspruch 3, **dadurch gekennzeichnet, dass** das Modellmaterial eine lineare Expansion von mindestens 0.5 % aufweist.

5. Verfahren nach Anspruch 4, **dadurch gekennzeichnet, dass** das Modellmaterial eine lineare Expansion von 4 % bis 12 % aufweist.

6. Verfahren nach Anspruch 5, **dadurch gekennzeichnet, dass** das Modellmaterial eine lineare Expansion von 8 % bis 10 % aufweist.

7. Verfahren nach einem der Ansprüche 3 bis 6, **dadurch gekennzeichnet, dass** es sich bei dem Modellmaterial um einen sogenannten Dentalgips handelt.

8. Verfahren nach einem der vorhergehenden Ansprüche, **dadurch gekennzeichnet, dass** bei der Bildung des Grünkörpers mindestens zwei, vorzugsweise alle Glieder des Dentalformteils in einem Arbeitsschritt gleichzeitig aus den keramischen Partikeln geformt werden.

9. Verfahren nach einem der vorhergehenden Ansprüche, **dadurch gekennzeichnet, dass** die den Pfeilern entsprechenden Teile (3, 4) des Modells (11) eine käppchenartige Form besitzen.

10. Verfahren nach einem der vorhergehenden Ansprüche, **dadurch gekennzeichnet, dass** die den Seitengliedern oder Brückengliedern entsprechenden Teile des Modells als Hohlform (7) ausgebildet sind.

11. Verfahren nach Anspruch 10, **dadurch gekennzeichnet, dass** die Hohlform (7) an den den Pfeilern entsprechenden Teilen (3, 4) des Modells befestigt ist.

12. Verfahren nach einem der Ansprüche 9 bis 11, **dadurch gekennzeichnet, dass** die den Pfeilern entsprechenden Teile des Modells aus einem Dentalgips mit erhöhter linearer Abbindeexpansion gefertigt sind.

13. Verfahren nach einem der Ansprüche 10 bis 12, **dadurch gekennzeichnet, dass** die Hohlform aus einem zahntechnisch modellierbaren Material gefertigt ist.

14. Verfahren nach einem der Ansprüche 10 bis 13, **dadurch gekennzeichnet, daß** die Hohlform dreischalig aufgebaut ist.

15. Verfahren nach Anspruch 14, **dadurch gekennzeichnet, dass** eine Unterschale und zwei die Hohlform nach oben abschließende Seitenschalen vorgesehen sind.

16. Verfahren nach einem der Ansprüche 10 bis 15, **dadurch gekennzeichnet, dass** die Hohlform an den den Pfeilern entsprechenden Teilen des Modells mit Hilfe eines zahntechnisch modellierbaren Materials befestigt ist.

17. Verfahren nach einem der vorhergehenden Ansprüche, **dadurch gekennzeichnet, dass** zur Herstellung eines Gerüstelements für Brücken
a) zwischen zwei den Pfeilern entsprechenden Teilen (3, 4) des Arbeitsmodells (1) ein Brückenglied (5) oder das Modell eines Brückenglieds plaziert und einmodelliert wird,
b) das Brückenglied (5) oder sein Modell einschließlich der Verbindungsstellen zu den Pfeilerteilen mit einem zahntechnisch modellierbaren Material von basal bis zum anatomischen Äquator einmodelliert wird,
c) die gemäß b) erhaltene Modellation (6) abgenommen und das Brückenglied (5) oder sein Modell vom Arbeitsmodell (1) entfernt wird,
d) die gemäß b) erhaltene Modellation (6) wieder in der Ursprungsposition auf dem Arbeitsmodell (1) plaziert und fixiert wird,
e) die fixierte Modellation (6) zu einer vollständigen Hohlform (7) aufmodelliert wird, und
f) mit Hilfe des so erhaltenen Modells von Pfeilern und Brückenglied und einer Suspension keramischer Partikel der genannte keramische Grünkörper (21) durch elektrophoretische Abscheidung gebildet wird.

18. Vorrichtung zur elektrophoretischen Abscheidung von zwei- oder mehrgliedrigen vollkeramischen Dentalformteilen, wie von Gerüstelementen für Brücken und dergleichen, **dadurch gekennzeichnet, dass** sie neben üblichen Komponenten wie Strom-/ Spannungsquelle, Steuer- und Regeleinrichtung, Abscheidebehältnis, Gegenelektrode, Kontaktierungseinrichtungen und dergleichen mindestens eine schaltbare Hilfselektrode aufweist, die an dem für die Abscheidung verwendeten Modell in der Nähe einer Verbindungsstelle zwischen Pfeiler und Seitenglied bzw. Brückenglied anordenbar ist.

19. Vorrichtung nach Anspruch 18, **dadurch gekennzeichnet, dass** zwei Hilfselektroden vorgesehen sind, die in der Nähe der Verbindungsstellen im Interdentalraum zwischen den Brückenpfeilern anordenbar sind.

20. Vorrichtung nach Anspruch 18 oder Anspruch 19, **dadurch gekennzeichnet, dass** die Hilfselektroden die entsprechenden Verbindungsstellen mindestens teilweise umschließen.

## Claims

1. Method for producing two-membered or multi-membered all-ceramic dental shaped parts, such as framework elements for bridges (31) and the like, in which method a model (11) is produced from the basic structure for which the dental shaped part is intended, and, with the aid of the model (11) and a suspension of ceramic particles, a ceramic green body (21) is formed, and the ceramic green body (21) is sintered, if appropriate after removal from the model (11), the green body (21) being formed from the ceramic particles by electrophoretic deposition and at least some of the dimensions of the model being selected so that the shrinkage which occurs during sintering of the green body is compensated, in order to achieve the desired fit between dental shaped part and basic structure.

2. Method according to Claim 1, **characterized in that** substantially all dimensions of the model (11) are selected so that the sintering shrinkage is compensated.

3. Method according to Claim 1 or Claim 2, **characterized in that**, in order to compensate for the sintering shrinkage, the model is made of a modelling material with a high linear setting expansion.

4. Method according to Claim 3, **characterized in that** the modelling material has a linear expansion of at least 0.5%.

5. Method according to Claim 4, **characterized in that** the modelling material has a linear expansion of from 4% to 12%.

6. Method according to Claim 5, **characterized in that** the modelling material has a linear expansion of from 8% to 10%.

7. Method according to Claims 3 to 6, **characterized in that** the modelling material is what is called dental plaster.

8. Method according to one of the preceding claims, **characterized in that** upon formation of the green body, at least two members, preferably all the members, of the dental shaped part are formed simultaneously from the ceramic particles in one work step.

9. Method according to one of the preceding claims, **characterized in that** the parts (3, 4) of the model (11) which correspond to the abutments have the shape of a cap.

10. Method according to one of the preceding claims, **characterized in that** the parts of the model which correspond to the side members or bridge members are designed as a hollow mould (7).

11. Method according to Claim 10, **characterized in that** the hollow mould (7) is secured to the parts (3, 4) of the model which correspond to the abutments.

12. Method according to one of Claims 9 to 11, **characterized in that** the parts of the model which correspond to the abutments are made of a dental plaster with a high linear setting expansion.

13. Method according to one of Claims 10 to 12, **characterized in that** the hollow mould is made of a dental modelling material.

14. Method according to one of Claims 10 to 13, **characterized in that** the hollow mould is made up of three shells.

15. Method according to Claim 14, **characterized in that** a bottom shell and two side shells closing the hollow mould at the top are provided.

16. Method according to one of Claims 10 to 15, **characterized in that** the hollow mould is secured to the parts of the model corresponding to the abutments with the aid of a dental modelling material.

17. Method according to one of the preceding claims, **characterized in that**, in order to produce a framework element for bridges,
a) a bridge member (5), or the model of a bridge member, is placed and modelled-in between two parts (3, 4) of the work model (1) which correspond to the abutments,
b) the bridge member (5) or its model, including the points of connection to the abutment parts, is modelled-in with a dental modelling material from the base up to the anatomical equator,
c) the modelled element (6) obtained in accordance with b) is taken off and the bridge member (5) or its model is removed from the work model (1),
d) the modelled element (6) obtained in accordance with b) is once again placed in the original position on the work model (1) and fixed,
e) the fixed modelled element (6) is shaped to give a complete hollow mould (7), and
f) said ceramic green body (21) is formed with the aid of the resulting model of abutments and bridge member and with the aid of a suspension of ceramic particles by electrophoretic deposition.

18. Device for electrophoretic deposition of two-membered or multi-membered all-ceramic dental shaped parts, such as framework elements for bridges and the like, **characterized in that**, in addition to customary components such as a current/voltage source, controlling and regulating means, deposition vessel, counterelectrode, contact-making arrangements and the like, it has at least one switchable auxiliary electrode which can be arranged on the model used for the deposition near a connection point between abutment and side member/bridge member.

19. Device according to Claim 18, **characterized in that** two auxiliary electrodes are provided which can be arranged near the connection points in the interdental space between the bridge abutments.

20. Device according to Claim 18 or Claim 19, **characterized in that** the auxiliary electrodes at least partially surround the corresponding connection points.

## Revendications

1. Procédé de fabrication de pièces dentaires en deux ou plusieurs parties, moulées en céramique pleine, par exemple des éléments d'ossature pour bridge (31) et similaires, dans lequel on prépare un moulage (11) de la structure de base pour laquelle la pièce dentaire moulée est prévue, on forme à l'aide du moulage (11) et d'une suspension de particules céramiques un corps cru (21) en céramique, on fritte le corps cru (21) en céramique, éventuellement après l'avoir enlevé du moulage (11), le corps cru (21) étant formé à partir des particules céramiques par dépôt électrophorétique et au moins certaines des dimensions du moulage étant sélectionnées de telle sorte que le retrait qui survient lors du frittage du corps cru soit compensé pour obtenir l'adaptation souhaitée entre la pièce dentaire moulée et la structure de base.

2. Procédé selon la revendication 1, **caractérisé en ce que** l'on sélectionne essentiellement toutes les dimensions du moulage (11) de manière à compenser le retrait au frittage.

3. Procédé selon la revendication 1 ou la revendication 2, **caractérisé en ce que** pour compenser le retrait au frittage, le moulage est réalisé en un matériau de moulage à plus forte dilatation linéaire lors de la prise.

4. Procédé selon la revendication 3, **caractérisé en ce que** le matériel de moulage présente une dilatation linéaire d'au moins 0,5 %.

5. Procédé selon la revendication 4, **caractérisé en ce que** le matériel de moulage présente une dilatation linéaire de 4 % à 12 %.

6. Procédé selon la revendication 5, **caractérisé en ce que** le matériau de moulage présente une dilatation linéaire de 8 % à 10 %.

7. Procédé selon l'une des revendications 3 à 6, **caractérisé en ce que** le matériau de moulage est un plâtre dit dentaire.

8. Procédé selon l'une des revendications précédentes, **caractérisé en ce que** lors de la formation du corps cru, au moins deux et de préférence toutes les parties de la pièce dentaire moulée sont formées simultanément en une étape de travail à partir des particules de céramique.

9. Procédé selon l'une des revendications précédentes, **caractérisé en ce que** les parties (3, 4) du moulage (11) qui correspondent aux piliers ont une forme en capuchon.

10. Procédé selon l'une des revendications précédentes, **caractérisé en ce que** les parties du moulage qui correspondent aux parties latérales ou aux parties de pontage sont configurées en moule creux (7).

11. Procédé selon la revendication 10, **caractérisé en ce que** le moule creux (7) est fixé sur les parties (3, 4) du moulage qui correspondent aux piliers.

12. Procédé selon l'une des revendications 9 à 11, **caractérisé en ce que** les parties du moulage qui correspondent aux piliers sont réalisées en plâtre dentaire à plus fort coefficient de dilatation à la prise.

13. Procédé selon l'une des revendications 10 à 12, **caractérisé en ce que** le moule creux est réalisé en un matériau moulable de la technique dentaire.

14. Procédé selon l'une des revendications 10 à 13, **caractérisé en ce que** le moule creux est constitué de trois coques.

15. Procédé selon la revendication 14, **caractérisé en ce qu'**une coque inférieure et deux coques latérales qui ferment le moule creux vers le haut sont prévues.

16. Procédé selon l'une des revendications 10 à 15, **caractérisé en ce que** le moule creux est fixé sur les parties du moulage qui correspondent aux piliers, à l'aide d'un matériau moulable de la technique dentaire.

17. Procédé selon l'une des revendications précédentes, **caractérisé en ce que** pour préparer un élément d'ossature pour bridge :
a) entre deux parties (3, 4) du moulage de travail (1) qui correspondent aux piliers, on place ou on moule un élément de pontage (5) ou le moulage d'un élément de pontage,
b) on moule l'élément de pontage (5) ou son moulage, y compris les emplacements de liaison aux parties en pilier, à l'aide d'un matériau moulable de la technique dentaire, depuis la base jusqu'à l'équateur anatomique.
c) le moulage (6) obtenu au point b) est enlevé et l'élément de pontage (5) ou son moulage est enlevé du moulage de travail (1),
d) le moulage (6) obtenu au point b) est replacé et fixé dans sa position initiale sur le modèle de travail (1),
e) le moulage (6) fixé est moulé de manière à obtenir un moule (7) complet et
f) à l'aide du moulage ainsi obtenu des piliers et de l'élément de pontage et d'une suspension de particules céramiques, on forme ledit corps cru (21) en céramique par dépôt électrophorétique.

18. Dispositif de dépôt électrophorétique de pièces dentaires moulées en céramique pleine en deux ou plusieurs éléments, par exemple d'éléments d'ossature de bridge ou similaires,
**caractérisé en ce que**
en plus des autres composants tels qu'une source de courant et/ou de tension, un dispositif de commande et de régulation, un récipient de dépôt, une contre-électrode, des dispositifs de mise contact et similaires, il présente au moins une électrode auxiliaire commutable qui peut être disposé sur le moulage utilisé pour le dépôt, à proximité d'un emplacement qui relie un pilier et un élément latéral ou un élément de pontage.

19. Dispositif selon la revendication 18, **caractérisé en ce qu'**il prévoit deux électrodes auxiliaires qui peuvent être disposées à proximité des emplacements de liaison dans l'espace interdentaire entre les piliers du pontage.

20. Dispositif selon la revendication 18 ou la revendication 19, **caractérisé en ce que** les électrodes auxiliaires entourent au moins en partie les emplacements de liaison respectifs.
